# EUROPEAN PATENT SPECIFICATION

(11) **EP 3 387 115 B1**
(45) Date of publication and mention of the grant of the patent: **26.01.2022**
(21) Application number: 16808635.3
(22) Date of filing: 08.12.2016
(51) Int. Cl.: A61P 31/04, A61P 31/10, A61P 31/12, A61P 35/00, C12N 5/0786

(54) **METHODS FOR EXPANDING A POPULATION OF ALVEOLAR MACROPHAGES IN A LONG TERM CULTURE**
VERFAHREN ZUR AUSDEHNUNG EINER POPULATION VON ALVEOLAREN MAKROPHAGEN IN EINER LANGZEITZÜCHTUNG
PROCÉDÉS PERMETTANT D'AUGMENTER UNE POPULATION DE MACROPHAGES ALVÉOLAIRES DANS UNE CULTURE À LONG TERME

(30) Priority: 08.12.2015 EP 15306959
(43) Date of publication of application: 17.10.2018
(73) Proprietor: INSERM (Institut National de la Santé et de la Recherche Médicale), 75013 Paris (FR); Université d'Aix Marseille, 13284 Marseille Cedex 07 (FR); Centre National de la Recherche Scientifique (CNRS), 75016 Paris (FR)
(72) Inventor: SIEWEKE, Michael, 13288 Marseille Cedex 09 (FR); MOLAWI, Kaaweh, 10707 Berlin (DE); GEIRSDOTTIR, Laufey, 79106 Freiburg (DE)
(74) Representative: Inserm Transfert
(86) International application number: PCT/EP2016/080187
(87) International publication number: WO 2017/097876

(56) References cited:
- EP-A1- 1 547 615
- H S LIN ET AL: "BOTH GRANULOCYTE-MACROPHAGE CSF AND MACROPHAGE CSF CONTROL THE PROLIFERATION AND SURVIVAL OF THE SAME SUBSET OF ALVEOLAR MACROPHAGES.", THE JOURNAL OF IMMUNOLOGY, vol. 142, no. 2, 15 January 1989 (1989-01-15), pages 515-519, XP055269308,
- KOH NAKATA ET AL: "Growth inhibitory factor of human alveolar macrophage", BIOCHEMICAL AND BIOPHYSICAL RESEARCH COMMUNICATIONS, vol. 180, no. 3, 1 November 1991 (1991-11-01), pages 1207-1213, XP055269311, US ISSN: 0006-291X, DOI: 10.1016/S0006-291X(05)81324-5
- ERIC CAREAU ET AL: "Adoptive Transfer of Alveolar Macrophages Abrogates Bronchial Hyperresponsiveness", AMERICAN JOURNAL OF RESPIRATORY CELL AND MOLECULAR BIOLOGY., vol. 31, no. 1, 1 July 2004 (2004-07-01), pages 22-27, XP055283720, NEW YORK, NY, US ISSN: 1044-1549, DOI: 10.1165/rcmb.2003-0229OC
- AL-HALLAK K M ET AL: "Secondary cytotoxicity mediated by alveolar macrophages: A contribution to the total efficacy of nanoparticles in lung cancer therapy?", EUROPEAN JOURNAL OF PHARMACEUTICS AND BIOPHARMACEUTICS, ELSEVIER SCIENCE PUBLISHERS B.V., AMSTERDAM, NL, vol. 76, no. 1, 1 September 2010 (2010-09-01), pages 112-119, XP027210089, ISSN: 0939-6411 [retrieved on 2010-08-12]

## Description

### FIELD:

The present disclosure relates to methods for expanding a population of alveolar macrophages in a long term culture.

### BACKGROUND:

In many tissues of the body, differentiated cells are frequently replaced as part of homeostatic maintenance or in response to injury. Whereas in most cases this depends on tissue-specific stem cells, tissue macrophages can be maintained by local proliferation independently of hematopoietic stem cells (1-4), possibly by self-renewal mechanisms activated in mature macrophages (5). Unlike the few examples of differentiated normal cells that can transiently reenter the cell cycle, such as hepatocytes, macrophages can also be expanded and maintained in long-term culture without transformation or loss of differentiation. This has been observed in macrophages with deletions of two core macrophage transcription factors (6), MafB and c-Maf (Maf-DKO macrophages) (7), or in cultures derived from fetal progenitors (8).

Lin HS. et al. disclose the effects of GM-CSF on alveolar macrophages on growth, survival and colony formation over several days (14 days) (Lin, H. S., Balakrishna L. Lokeshwar, and S. H. I. N. Hsu. "Both granulocyte-macrophage CSF and macrophage CSF control the proliferation and survival of the same subset of alveolar macrophages." The Journal of Immunology 142.2 (1989): 515-519).

Nakata, Koh, et al. disclose the cultivation of human alveolar macrophages in medium supplement with GM-CSF for several days (Nakata, Koh, et al. "Growth inhibitory factor of human alveolar macrophage." Biochemical and biophysical research communications 180.3 (1991): 1207-1213).

Careau E. et al. disclose the use of alveolar macrophages in modulating inflammatory disorders of the lung. (Careau, Eric, and Elyse Y. Bissonnette. "Adoptive transfer of alveolar macrophages abrogates bronchial hyperresponsiveness." American journal of respiratory cell and molecular biology 31.1 (2004): 22-27).

Al-Hallak, Kamal MHD, et al. disclose the use of alveolar macrophages in lung cancer therapy (Al-Hallak, Kamal MHD, et al. "Secondary cytotoxicity mediated by alveolar macrophages: a contribution to the total efficacy of nanoparticles in lung cancer therapy?." European Journal of Pharmaceutics and Biopharmaceutics 76.1 (2010): 112-119).

EP1547615 discloses the use of alveolar macrophages as therapeutic for lung cancer.

### SUMMARY OF THE INVENTION:

The present invention is defined by the claims. In particular, the present invention relates to a method of expanding a population of alveolar macrophages in a long term liquid culture comprising culturing for at least 100 days the population of alveolar macrophages in a culture medium supplemented with an amount of GM-CSF.

### DETAILED DESCRIPTION:

An object of the present disclosure relates to a method of expanding a population of alveolar macrophages in a long term culture comprising culturing the population of alveolar macrophages in a culture medium supplemented with an amount of GM-CSF.

As used herein, the term "alveolar macrophage" has its general meaning in the art and refers to a specific subset of macrophages that is present in the lung alveoli of a mammal. Typically, the mammal is selected from the group consisting of rodents (e.g. mice or rats), dogs, pigs, cats, horses, cows, sheep, camels or primates and humans. Methods for obtaining a population of alveolar macrophages from a mammal are conventional and typically include bronchial lavage. According to the present disclosure, the alveolar macrophages are self-renewing. As used herein, the term "self-renewing" has its general meaning in the art and refers to the ability the alveolar macrophages to go through numerous cycles of cell division giving rise to more alveolar macrophages of the same or highly similar differentiated cellular phenotype. Typically, the self- renewing alveolar macrophages maintain their ability to divide for an unlimited amount or extended period of time.

As used herein, the term "expanding" has its general meaning in the art and refers to a multiplication of cells, thus resulting in an increase in the total number of alvelolar macrophages. For example, the alveolar macrophages can be expanded to at least 100 times their original number, such as at least 1.000 times their original number, preferably at least 10.000 times their original number, such as at least 100.000 times their original number etc..

According to the present disclosure, the method of the present disclosure thus not only allows the expansion of the alveolar macrophages but also allows the alveolar macrophages to maintain their phenotype in a long term manner. Such phenotype includes, without being limiting, the biological function or and specific marker expression profile. Non-limiting examples of the biological functions include their function as phagocytes, the ability for killing of microorganisms, the ability to produce inflammatory mediators such as cytokines, chemokines, growth factors and immunological mediators such as interferons and to upregulate the expression of surface markers (CD69, CD14) important in the activation of innate immune cells in response to stimulation. The specific marker expression profile includes the presence of e.g. GM-CSF receptor, M-CSF receptor, CD11c, TLRs, F4/80, CD16, CD11b, CD14 and/or Marco and/or a lack of expression of MHC-II, B220, Gr1 , IgE receptor, T-cell receptor and/or B-cell receptor, as defined herein below.

Typically, the culture medium of the present disclosure corresponds to any culture medium that is conventional for culturing the population of alveolar macrophages of the present disclosure. For example, the culture medium can be a medium selected from the group consisting of RPMI 1640, Iscove's, F12, OPTI-MEM, DMEM, etc... In particular, the medium is supplemented with serum, including non- human or human serum. Serum such as for example FCS may be obtained from e.g. GIBCO, Sigma or PAA. According to the present disclosure the culture medium is thus supplemented with an amount of GM-CSF. As used herein the term ""GM-CSF" has its general meaning in the art and refers to to granulocyte- macrophage colony-stimulating factor. GM-CSF is a cytokine that acts as a growth factor for leukocytes. An exemplary human amino acid sequence is represented by the GenBank data base entry AAA52578.1. Typically, the GM-CSF is a recombinantly produced and various commercial sources are available. The method herein disclosed is carried out under suitable cell culture conditions. General cell culture conditions as well as suitable cell culture media are well known in the art. Typically, the cell culture conditions comprise conditions of about 2 to 10% CO2, preferably about 5% CO2 and a temperature of about 32 to 38°C, typically about 37°C.

The population of alveolar macrophages as prepared by the method of the present disclosure may find various applications. In particular, the population of the alveolar macrophages may be used for the treatment of various diseases. For therapeutic application, the skilled artisan could imagine that the method of the present disclosure is suitable for preparing huge amounts of alveolar macrophages obtained from a patient that can be re-administered to the patient for the treatment of a disease. In that case the transplantation is autologous. But in particular, the transplantation can also be non-autologous. For non-autologous transplantation, the recipient will be screened for a maximal match of MHC haplotype and/or is preferably given an immunosuppressive drug to reduce the risk of rejection of the transplanted cell. Methods of administering the population of alveolar macrophages herein disclosed include but are not limited to intradermal, intramuscular, intraperitoneal, intravenous, subcutaneous, intranasal, intrapulmonary, intra-tracheal and epidural routes. In particular, the population of alveolar macrophages is administered in to the pulmonary system of the patient (e.g. by lavage). Thus, the population of alveolar macrophages of the present disclosure is suitable for pulmonary macrophage transplantation therapy. The cells may be administered by any convenient route, and may be administered together with other biologically active agents. The titer of the cells transplanted which will be effective in the treatment of a particular disease or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each subject's circumstances.

The method of the present disclosure is thus particularly suitable for preparing a pharmaceutical composition comprising large amount alveolar macrophages. In particular, the method of the present disclosure is particularly suitable for preparing a pharmaceutical composition comprising at least 1 ×10⁹- 1 ×10¹² of alveolar macrophages. In particular, the pharmaceutical composition comprises 1, 2, 3, 4, 5, 6, 7, 8, 9, 10, 15, 20, 25, 50, 75, 100, 150, 200, 250, 300, 350, 400, 450, 500, 550, 600, 650, 700, 750, 800, 850, 900, 950, or 1 trillion of alveolar macrophages. In particular, the methods herein disclosed provide a substantially homogeneous population of functionally differentiated alveolar macrophages. The term "substantially homogeneous population", as used herein, refers to a population of cells wherein the majority (e.g., at least about 80%, preferably at least about 90%, more preferably at least about 95%) of the total number of cells have the specific characteristics of the fully differentiated alveolar macrophages. Typically the pharmaceutical composition comprises a pharmaceutically acceptable carrier or excipient. As used herein, the term "pharmaceutically acceptable carrier or excipient" refers to a carrier medium which does not interfere with the effectiveness of the biological activity of the functionally differentiated somatic cells herein disclosed, and which is not excessively toxic to the host at the concentrations at which it is administered. Examples of suitable pharmaceutically acceptable carriers or excipients include, but are not limited to, water, salt solution (e.g., Ringer's solution), alcohols, oils, gelatins, carbohydrates (e.g., lactose, amylase or starch), fatty acid esters, hydroxymethylcellulose, and polyvinyl pyroline. Pharmaceutical compositions may be formulated as liquids, semi-liquids (e.g., gels) or solids (e.g., matrix, lattices, scaffolds, and the like).

In particular, the population of alveolar macrophages as prepared by the method of the present disclosure is particularly suitable for the treatment of cancer. As used herein, the term "cancer" has its general meaning in the art and includes, but is not limited to, solid tumors and blood borne tumors. The term cancer includes diseases of the skin, tissues, organs, bone, cartilage, blood and vessels. The term "cancer" further encompasses both primary and metastatic cancers. Examples of cancers that may treated by methods and compositions herein disclosed include, but are not limited to, cancer cells from the bladder, blood, bone, bone marrow, brain, breast, colon, esophagus, gastrointestinal, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malign melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

In particular, the population of alveolar macrophages as prepared by the method of the present disclosure is particularly suitable for the treatment of an infectious disease. As used herein the term "infectious disease" includes any infection caused by viruses, bacteria, protozoa, molds or fungi. In particular, the viral infection comprises infection by one or more viruses selected from the group consisting of *Arenaviridae, Astroviridae, Birnaviridae, Bromoviridae, Bunyaviridae, Caliciviridae, Closteroviridae, Comoviridae, Cystoviridae, Flaviviridae, Flexiviridae, Hepevirus, Leviviridae, Luteoviridae, Mononegavirales, Mosaic Viruses, Nidovirales, Nodaviridae, Orthomyxoviridae, Picobirnavirus, Picornaviridae, Potyviridae, Reoviridae, Retroviridae, Sequiviridae, Tenuivirus, Togaviridae, Tombusviridae, Totiviridae, Tymoviridae, Hepadnaviridae, Herpesviridae, Paramyxoviridae or Papillomaviridae viruses. Relevant taxonomic families of RNA viruses include, without limitation, Astroviridae, Birnaviridae, Bromoviridae, Caliciviridae, Closteroviridae, Comoviridae, Cystoviridae, Flaviviridae, Flexiviridae, Hepevirus, Leviviridae, Luteoviridae, Mononegavirales, Mosaic Viruses, Nidovirales, Nodaviridae, Orthomyxoviridae, Picobirnavirus, Picornaviridae, Potyviridae, Reoviridae, Retroviridae, Sequiviridae, Tenuivirus, Togaviridae, Tombusviridae, Totiviridae,* and *Tymoviridae* viruses. In particular, the viral infection comprises infection by one or more viruses selected from the group consisting of adenovirus, rhinovirus, hepatitis, immunodeficiency virus, polio, measles, Ebola, Coxsackie, Rhino, West Nile, small pox, encephalitis, yellow fever, Dengue fever, influenza (including human, avian, and swine), lassa, lymphocytic choriomeningitis, junin, machuppo, guanarito, hantavirus, Rift Valley Fever, La Crosse, California encephalitis, Crimean-Congo, Marburg, Japanese Encephalitis, Kyasanur Forest, Venezuelan equine encephalitis, Eastern equine encephalitis, Western equine encephalitis, severe acute respiratory syndrome (SARS), parainfluenza, respiratory syncytial, Punta Toro, Tacaribe, pachindae viruses, adenovirus, Dengue fever, influenza A and influenza B (including human, avian, and swine), junin, measles, parainfluenza, Pichinde, punta toro, respiratory syncytial, rhinovirus, Rift Valley Fever, severe acute respiratory syndrome (SARS), Tacaribe, Venezuelan equine encephalitis, West Nile and yellow fever viruses, tick-borne encephalitis virus, Japanese encephalitis virus, St. Louis encephalitis virus, Murray Valley virus, Powassan virus, Rocio virus, louping-ill virus, Banzi virus, Ilheus virus, Kokobera virus, Kunjin virus, Alfuy virus, bovine diarrhea virus, and Kyasanur forest disease. Bacterial infections that can be treated according to the present disclosure include, but are not limited to, infections caused by the following: *Staphylococcus; Streptococcus,* including *S. pyogenes; Enterococcl; Bacillus,* including *Bacillus anthracis,* and *Lactobacillus; Listeria; Corynebacterium diphtheriae; Gardnerella* including *G. vaginalis; Nocardia; Streptomyces; Thermoactinomyces vulgaris; Treponerna; Camplyobacter, Pseudomonas* including *aeruginosa; Legionella; Neisseria including N.gonorrhoeae and N.meningitides; Flavobacterium* including *F. meningosepticum and F. odoraturn; Brucella; Bordetella* including *B. pertussis and B. bronchiseptica; Escherichia* including *E. coli, Klebsiella; Enterobacter, Serratia including S. marcescens* and *S. liquefaciens; Edwardsiella; Proteus* including *P. mirabilis* and *P. vulgaris; Streptobacillus; Rickettsiaceae* including R *fickettsfi, Chlamydia* including *C. psittaci and C. trachornatis; Mycobacterium* including *M. tuberculosis, M. intracellulare, M. folluiturn, M. laprae, M. avium, M. bovis, M. africanum, M. kansasii, M. intracellulare,* and *M. lepraernurium; and Nocardia.* Protozoa infections that may be treated according to the present disclosure include, but are not limited to, infections caused by leishmania, kokzidioa, and trypanosoma. A complete list of infectious diseases can be found on the website of the National Center for Infectious Disease (NCID) at the Center for Disease Control (CDC) (World Wide Web (www) at cdc.gov/ncidod/diseases/). All of said diseases are candidates for treatment using the compositions herein disclosed.

In particular, the population of alveolar macrophages as prepared by the method of the present disclosure is particularly suitable for the treatment of autoimmune and inflammatory disease. The autoimmune and inflammatory diseases may be one or more selected from the group consisting of arthritis, rheumatoid arthritis, acute arthritis, chronic rheumatoid arthritis, gouty arthritis, acute gouty arthritis, chronic inflammatory arthritis, degenerative arthritis, infectious arthritis, Lyme arthritis, proliferative arthritis, psoriatic arthritis, vertebral arthritis, and juvenile-onset rheumatoid arthritis, osteoarthritis, arthritis chronica progrediente, arthritis deformans, polyarthritis chronica primaria, reactive arthritis, and ankylosing spondylitis), inflammatory hyperproliferative skin diseases, psoriasis such as plaque psoriasis, gutatte psoriasis, pustular psoriasis, and psoriasis of the nails, dermatitis including contact dermatitis, chronic contact dermatitis, allergic dermatitis, allergic contact dermatitis, dermatitis herpetiformis, and atopic dermatitis, x-linked hyper IgM syndrome, urticaria such as chronic allergic urticaria and chronic idiopathic urticaria, including chronic autoimmune urticaria, polymyositis/dermatomyositis, juvenile dermatomyositis, toxic epidermal necrolysis, scleroderma, systemic scleroderma, sclerosis, systemic sclerosis, multiple sclerosis (MS), spino-optical MS, primary progressive MS (PPMS), relapsing remitting MS (RRMS), progressive systemic sclerosis, atherosclerosis, arteriosclerosis, sclerosis disseminata, and ataxic sclerosis, inflammatory bowel disease (IBD), Crohn's disease, colitis, ulcerative colitis, colitis ulcerosa, microscopic colitis, collagenous colitis, colitis polyposa, necrotizing enterocolitis, transmural colitis, autoimmune inflammatory bowel disease, pyoderma gangrenosum, erythema nodosum, primary sclerosing cholangitis, episcleritis, respiratory distress syndrome, adult or acute respiratory distress syndrome (ARDS), meningitis, inflammation of all or part of the uvea, iritis, choroiditis, an autoimmune hematological disorder, rheumatoid spondylitis, sudden hearing loss, IgE-mediated diseases such as anaphylaxis and allergic and atopic rhinitis, encephalitis, Rasmussen's encephalitis, limbic and/or brainstem encephalitis, uveitis, anterior uveitis, acute anterior uveitis, granulomatous uveitis, nongranulomatous uveitis, phacoantigenic uveitis, posterior uveitis, autoimmune uveitis, glomerulonephritis (GN), idiopathic membranous GN or idiopathic membranous nephropathy, membrano- or membranous proliferative GN (MPGN), rapidly progressive GN, allergic conditions, autoimmune myocarditis, leukocyte adhesion deficiency, systemic lupus erythematosus (SLE) or systemic lupus erythematodes such as cutaneous SLE, subacute cutaneous lupus erythematosus, neonatal lupus syndrome (NLE), lupus erythematosus disseminatus, lupus (including nephritis, cerebritis, pediatric, non-renal, extra-renal, discoid, alopecia), juvenile onset (Type I) diabetes mellitus, including pediatric insulin-dependent diabetes mellitus (IDDM), adult onset diabetes mellitus (Type II diabetes), autoimmune diabetes, idiopathic diabetes insipidus, immune responses associated with acute and delayed hypersensitivity mediated by cytokines and T-lymphocytes, tuberculosis, sarcoidosis, granulomatosis, lymphomatoid granulomatosis, Wegener's granulomatosis, agranulocytosis, vasculitides, including vasculitis, large vessel vasculitis, polymyalgia rheumatica, giant cell (Takayasu's) arteritis, medium vessel vasculitis, Kawasaki's disease, polyarteritis nodosa, microscopic polyarteritis, CNS vasculitis, necrotizing, cutaneous, hypersensitivity vasculitis, systemic necrotizing vasculitis, and ANCA-associated vasculitis, such as Churg-Strauss vasculitis or syndrome (CSS), temporal arteritis, aplastic anemia, autoimmune aplastic anemia, Coombs positive anemia, Diamond Blackfan anemia, hemolytic anemia or immune hemolytic anemia including autoimmune hemolytic anemia (AIHA), pernicious anemia (anemia perniciosa), Addison's disease, pure red cell anemia or aplasia (PRCA), Factor VIII deficiency, hemophilia A, autoimmune neutropenia, pancytopenia, leukopenia, diseases involving leukocyte diapedesis, CNS inflammatory disorders, multiple organ injury syndrome such as those secondary to septicemia, trauma or hemorrhage, antigen-antibody complex-mediated diseases, anti-glomerular basement membrane disease, anti-phospholipid antibody syndrome, allergic neuritis, Bechet's or Behcet's disease, Castleman's syndrome, Goodpasture's syndrome, Reynaud's syndrome, Sjogren's syndrome, Stevens-Johnson syndrome, pemphigoid such as pemphigoid bullous and skin pemphigoid, pemphigus, optionally pemphigus vulgaris, pemphigus foliaceus, pemphigus mucus-membrane pemphigoid, pemphigus erythematosus, autoimmune polyendocrinopathies, Reiter's disease or syndrome, immune complex nephritis, antibody-mediated nephritis, neuromyelitis optica, polyneuropathies, chronic neuropathy, IgM polyneuropathies, IgM-mediated neuropathy, thrombocytopenia, thrombotic thrombocytopenic purpura (TTP), idiopathic thrombocytopenic purpura (ITP), autoimmune orchitis and oophoritis, primary hypothyroidism, hypoparathyroidism, autoimmune thyroiditis, Hashimoto's disease, chronic thyroiditis (Hashimoto's thyroiditis); subacute thyroiditis, autoimmune thyroid disease, idiopathic hypothyroidism, Grave's disease, polyglandular syndromes such as autoimmune polyglandular syndromes (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis, allergic encephalomyelitis, experimental allergic encephalomyelitis (EAE), myasthenia gravis, thymoma-associated myasthenia gravis, cerebellar degeneration, neuromyotonia, opsoclonus or opsoclonus myoclonus syndrome (OMS), and sensory neuropathy, multifocal motor neuropathy, Sheehan's syndrome, autoimmune hepatitis, chronic hepatitis, lupoid hepatitis, giant cell hepatitis, chronic active hepatitis or autoimmune chronic active hepatitis, lymphoid interstitial pneumonitis, bronchiolitis obliterans (non-transplant) vs NSIP, Guillain-Barre syndrome, Berger's disease (IgA nephropathy), idiopathic IgA nephropathy, linear IgA dermatosis, primary biliary cirrhosis, pneumonocirrhosis, autoimmune enteropathy syndrome, Celiac disease, Coeliac disease, celiac sprue (gluten enteropathy), refractory sprue, idiopathic sprue, cryoglobulinemia, amylotrophic lateral sclerosis (ALS; Lou Gehrig's disease), coronary artery disease, autoimmune ear disease such as autoimmune inner ear disease (AGED), autoimmune hearing loss, opsoclonus myoclonus syndrome (OMS), polychondritis such as refractory or relapsed polychondritis, pulmonary alveolar proteinosis, amyloidosis, scleritis, a non-cancerous lymphocytosis, a primary lymphocytosis, which includes monoclonal B cell lymphocytosis, optionally benign monoclonal gammopathy or monoclonal garnmopathy of undetermined significance, MGUS, peripheral neuropathy, paraneoplastic syndrome, channelopathies such as epilepsy, migraine, arrhythmia, muscular disorders, deafness, blindness, periodic paralysis, and channelopathies of the CNS, autism, inflammatory myopathy, focal segmental glomerulosclerosis (FSGS), endocrine opthalmopathy, uveoretinitis, chorioretinitis, autoimmune hepatological disorder, fibromyalgia, multiple endocrine failure, Schmidt's syndrome, adrenalitis, gastric atrophy, presenile dementia, demyelinating diseases such as autoimmune demyelinating diseases, diabetic nephropathy, Dressler's syndrome, alopecia greata, CREST syndrome (calcinosis, Raynaud's phenomenon, esophageal dysmotility, sclerodactyl), and telangiectasia), male and female autoimmune infertility, mixed connective tissue disease, Chagas' disease, rheumatic fever, recurrent abortion, farmer's lung, erythema multiforme, post-cardiotomy syndrome, Cushing's syndrome, bird-fancier's lung, allergic granulomatous angiitis, benign lymphocytic angiitis, Alport's syndrome, alveolitis such as allergic alveolitis and fibrosing alveolitis, interstitial lung disease, transfusion reaction, leprosy, malaria, leishmaniasis, kypanosomiasis, schistosomiasis, ascariasis, aspergillosis, Sampter's syndrome, Caplan's syndrome, dengue, endocarditis, endomyocardial fibrosis, diffuse interstitial pulmonary fibrosis, interstitial lung fibrosis, idiopathic pulmonary fibrosis, cystic fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, iridocyclitis, or Fuch's cyclitis, Henoch-Schonlein purpura, human immunodeficiency virus (HIV) infection, echovirus infection, cardiomyopathy, Alzheimer's disease, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, chorioiditis, giant cell polymyalgia, endocrine ophthamopathy, chronic hypersensitivity pneumonitis, keratoconjunctivitis sicca, epidemic keratoconjunctivitis, idiopathic nephritic syndrome, minimal change nephropathy, benign familial and ischemia-reperfusion injury, retinal autoimmunity, joint inflammation, bronchitis, chronic obstructive airway disease, silicosis, aphthae, aphthous stomatitis, arteriosclerotic disorders, aspermiogenese, autoimmune hemolysis, Boeck's disease, cryoglobulinemia, Dupuytren's contracture, endophthalmia phacoanaphylactica, enteritis allergica, erythema nodosum leprosum, idiopathic facial paralysis, chronic fatigue syndrome, febris rheumatica, Hamman-Rich's disease, sensoneural hearing loss, haemoglobinuria paroxysmatica, hypogonadism, ileitis regionalis, leucopenia, mononucleosis infectiosa, traverse myelitis, primary idiopathic myxedema, nephrosis, ophthalmia symphatica, orchitis granulomatosa, pancreatitis, polyradiculitis acuta, pyoderma gangrenosum, Quervain's thyreoiditis, acquired splenic atrophy, infertility due to antispermatozoan antobodies, non-malignant thymoma, vitiligo, SCID and Epstein-Barr virus-associated diseases, acquired immune deficiency syndrome (AIDS), parasitic diseases such as Lesihmania, toxic-shock syndrome, food poisoning, conditions involving infiltration of T cells, leukocyte-adhesion deficiency, immune responses associated with acute and delayed hypersensitivity mediated by cytokines and T-lymphocytes, diseases involving leukocyte diapedesis, multiple organ injury syndrome, antigen-antibody complex-mediated diseases, antiglomerular basement membrane disease, allergic neuritis, autoimmune polyendocrinopathies, oophoritis, primary myxedema, autoimmune atrophic gastritis, sympathetic ophthalmia, rheumatic diseases, mixed connective tissue disease, nephrotic syndrome, insulitis, polyendocrine failure, peripheral neuropathy, autoimmune polyglandular syndrome type I, adult-onset idiopathic hypoparathyroidism (AOIH), alopecia totalis, dilated cardiomyopathy, epidermolisis bullosa acquisita (EBA), hemochromatosis, myocarditis, nephrotic syndrome, primary sclerosing cholangitis, purulent or nonpurulent sinusitis, acute or chronic sinusitis, ethmoid, frontal, maxillary, or sphenoid sinusitis, an eosinophil-related disorder such as eosinophilia, pulmonary infiltration eosinophilia, eosinophilia-myalgia syndrome, Loffler's syndrome, chronic eosinophilic pneumonia, tropical pulmonary eosinophilia, bronchopneumonic aspergillosis, aspergilloma, or granulomas containing eosinophils, anaphylaxis, seronegative spondyloarthritides, polyendocrine autoimmune disease, sclerosing cholangitis, sclera, episclera, chronic mucocutaneous candidiasis, Bruton's syndrome, transient hypogammaglobulinemia of infancy, Wiskott-Aldrich syndrome, ataxia telangiectasia, autoimmune disorders associated with collagen disease, rheumatism, neurological disease, ischemic re-perfusion disorder, reduction in blood pressure response, vascular dysfunction, antgiectasis, tissue injury, cardiovascular ischemia, hyperalgesia, cerebral ischemia, and disease accompanying vascularization, allergic hypersensitivity disorders, glomerulonephritides, reperfusion injury, reperfusion injury of myocardial or other tissues, dermatoses with acute inflammatory components, acute purulent meningitis or other central nervous system inflammatory disorders, ocular and orbital inflammatory disorders, granulocyte transfusion-associated syndromes, cytokine-induced toxicity, acute serious inflammation, chronic intractable inflammation, pyelitis, pneumonocirrhosis, diabetic retinopathy, diabetic large-artery disorder, endarterial hyperplasia, peptic ulcer, valvulitis, and endometriosis.

In particular, the population of alveolar macrophages as prepared by the method of the present disclosure is particularly suitable for the treatment of Pulmonary Alveolar Proteinosis (PAP).

As used herein, the term "Pulmonary Alveolar Proteinosis" or "PAP" has its general meaning in the art and refers to a lung disease in which abnormal accumulation of pulmonary surfactant occurs within the alveoli, interfering with gas exchange. Congenital PAP is caused by mutations of the GM-CSF-R encoding genes *Csf2ra* or *Csf2rb* or GM-CSF encoding gene *csf2,* whereas the more frequent acquired form of the disease is caused by autoantibodies against GM-CSF (Trapnell, B.C. et al., T. Pulmonary alveolar proteinosis, a primary immunodeficiency of impaired GM-CSF stimulation of macrophages. Curr Opin Immunol 21, 514-521 (2009)).

In particular, the population of alveolar macrophages as prepared by the method of the present disclosure is particularly suitable for the treatment of degenerative disease or to stimulate regeneration of damaged tissue. The mononuclear phagocyte system (monocyte and macrophages) indeed represents a distributed organ responsible for homeostasis within the host. Said system is involved in every disease process in which there is persistent tissue injury or metabolic disturbance. Macrophages thus mediate acute as well as chronic inflammation, and promote repair through removal of dead cells and fibrin by phagocytosis and fibrinolysis, induce blood vessel ingrowth (angiogenesis) and modulate fibroblast invasion and production of extracellular matrix. They produce mediators that mobilize systemic responses of the host including fever, release and catabolize stress and other hormones, increase metabolic activity of other cells, and influence blood flow to tissues and capillary permeability. The macrophages themselves display considerable heterogeneity in their functions, often expressing activators as well as inhibitors of a property, e.g. proteolytic activity, or pro- and anti-inflammatory cytokine production, depending on the evolution of a particular host response. Furthermore, macrophages may inhibit precursor cells apoptosis in a cell to cell contact and may serve as stromal support for efficient cellular engraftment for tissue repair (see for example document WO2005014016). In particular macrophages could inhibit myogenic precursor cells apoptosis. Therefore methods for generating macrophages as above described may be useful for the treatment of lesions such as bone or muscular lesion, possibly resulting from a disease or an injury. It can be for example a bone fracture, or a torn muscle. In particular, said lesion is a cardiac lesion or injury. In particular, it can be for example myocardial infarction, heart insufficiency, coronary thrombosis, dilated cardiomyopathy or any cardiomyocyte dysfunction subsequent to, or resulting from, any genetic defect. Therefore, methods for generating macrophages as above described may be useful for the treatment of acute cardiac insufficiencies with bad prognostic despite progress in treatments, such as infiltrative cardiomyopathy, or cardiomyopathy due to anthracyclin toxicity or cardiomyopathy secondary to HIV infection. Methods for generating macrophages as above described may be also useful for the treatment of spinal cord injury. A therapy for complete spinal cord injury (SCI) may be indeed consisting in autologous grafts of macrophages that have been educated to a wound-healing phenotype by co-incubation with skin tissue. Macrophages are also essential for wound healing and thus methods for generating macrophages as above described may be useful for enhancing wound healing and/or repairing tissue damage. The wound healing process has indeed 3 phases. During the inflammatory phase, numerous enzymes and cytokines are secreted by the macrophage. These include collagenases, which clear the wound of debris, interleukins and tumor necrosis factor (TNF), which stimulate fibroblasts (to produce collagen) and promote angiogenesis; and transforming growth factor □ (TGF□), which stimulates keratinocytes. This step marks the transition into the process of tissue reconstruction, ie, the proliferative phase.

In particular, the population of alveolar macrophages as prepared by the method of the present disclosure is particularly suitable for the treatment of pulmonary diseases. For instance, the pulmonary disease is selected from the group consisting of asthma, asbestosis, silicosis, chronic obstructive pulmonary disease (COPD), (in particular exacerbation of COPD), neonatal respiratory distress, adult respiratory distress syndrome, congenital deficiencies of surfactant protein B, and allergic asthma. In particular, the population of alveolar macrophages as prepared by the method of the present disclosure is particularly suitable of preventing the increased susceptibility of some individuals to microbial challenge, especially in the setting of inadequate or impaired specific immunity. These disorders as well as some disorders associated with increased risk of pneumonia (cystic fibrosis, asthma, prematurity, chronic bronchitis, diffuse alveolar damage) may also be associated with acquired defects or deficiency in collectin function. In particular, the pulmonary disease is selected from the group consisting of cystic fibrosis, emphysema, infectious diseases, inflammatory diseases, and transplantation rejection. In particular, the pulmonary disease is an autoimmune or inflammatory disease and typically include pulmonary alveolar proteinosis, diffuse interstitial pulmonary fibrosis, interstitial lung fibrosis, and idiopathic pulmonary fibrosis. In particular, the pulmonary disease is a pulmonary infection. In particular, the pulmonary infection is bacterial, viral or fungal pneumonia In particular, the pulmonary viral disease is selected from the group consisting of influenza A, rhinovirus, coronavirus, Respiratory Syncytial Virus (RSV), chickenpox, human parvovirus B19, parainfluenza virus types 1-3, cytomegalovirus, adenovirus, hantavirus and rubella.

Moreover, the population of alveolar macrophages of the present disclosure is further genetically engineered so that said cells express a therapeutic nucleic acid of interest, which encodes a protein of interest. Suitable genes of interest include growth factors. For instance, population of alveolar macrophages of the present disclosure may be genetically engineered to produce gene products beneficial upon transplantation of the genetically engineered cells to a subject. Such gene products include, but are not limited to, anti-inflammatory factors, e.g., anti-TNF, anti-IL-1, anti- II-6, anti-IL-2...etc. The population of alveolar macrophages of the present disclosure may also be further genetically engineered so that said cells correct a genetic defect before retransplantation. Alternatively, the population of alveolar macrophages of the present disclosure may be fused to other functionally differentiated somatic cells to correct genetic defects in the target cell or to deliver therapeutic compounds. Indeed, macrophages have been shown to fuse with cardiac muscle cells or hepatocytes and may correct a genetic defect in these cells as described in Camargo et al., 2003; Camargo et al., 2004 and Willenbring et al., 2004. For example, cells herein disclosed such as macrophages may therefore be also engineered to express multiple or single copies of normal or hyperactive variants of genes that are mutated in genetic disorders. Examples include but are not limited to enzyme deficiencies in the liver or dystrophin in Duchenne muscular dystrophy.

In particular, the population of alveolar macrophages herein disclosed is further genetically engineered in order to downregulate one or more gene(s) of interest, such as MafB gene which has a negative effect on the proliferation/self renewal of macrophages.

In particular, the population of alveolar macrophages herein disclosed is genetically engineered with the "CRISPR/Cas9" technology.

As used herein, the term "CRISPR" has its general meaning in the art and refers to clustered regularly interspaced short palindromic repeats associated which are the segments of prokaryotic DNA containing short repetitions of base sequences. In bacteria the CRISPR/Cas loci encode RNA-guided adaptive immune systems against mobile genetic elements (viruses, transposable elements and conjugative plasmids). Three types (I-III) of CRISPR systems have been identified. CRISPR clusters contain spacers, the sequences complementary to antecedent mobile elements. CRISPR clusters are transcribed and processed into mature CRISPR (Clustered Regularly Interspaced Short Palindromic Repeats) RNA (crRNA). The CRISPR-associated endonuclease, Cas9, belongs to the type II CRISPR/Cas system and has strong endonuclease activity to cut target DNA. Cas9 is guided by a mature crRNA that contains about 20 base pairs (bp) of unique target sequence (called spacer) and a trans-activated small RNA (tracrRNA) that serves as a guide for ribonuclease Ill-aided processing of pre-crRNA. The crRNA:tracrRNA duplex directs Cas9 to target DNA via complementary base pairing between the spacer on the crRNA and the complementary sequence (called protospacer) on the target DNA. Cas9 recognizes a trinucleotide (NGG) protospacer adjacent motif (PAM) to specify the cut site (the 3rd nucleotide from PAM). The crRNA and tracrRNA can be expressed separately or engineered into an artificial fusion small guide RNA (sgRNA) via a synthetic stem loop to mimic the natural crRNA/tracrRNA duplex. Such sgRNA, like shRNA, can be synthesized or in vitro transcribed for direct RNA transfection or expressed from U6 or HI-promoted RNA expression vector, although cleavage efficiencies of the artificial sgRNA are lower than those for systems with the crRNA and tracrRNA expressed separately. In particular, the CRISPR-associated endonuclease can be a Cas9 nuclease. The Cas9 nuclease can have a nucleotide sequence identical to the wild type Streptococcus pyrogenes sequence. In particular, the CRISPR-associated endonuclease can be a sequence from other species, for example other Streptococcus species, such as thermophilus; Pseudomona aeruginosa, Escherichia coli, or other sequenced bacteria genomes and archaea, or other prokaryotic microogranisms. Alternatively, the wild type Streptococcus pyrogenes Cas9 sequence can be modified. The nucleic acid sequence can be codon optimized for efficient expression in mammalian cells, i.e., "humanized." A humanized Cas9 nuclease sequence can be for example, the Cas9 nuclease sequence encoded by any of the expression vectors listed in Genbank accession numbers KM099231.1 GL669193757; KM099232.1 GL669193761; or KM099233.1 GL669193765. Alternatively, the Cas9 nuclease sequence can be for example, the sequence contained within a commercially available vector such as PX330 or PX260 from Addgene (Cambridge, MA). In particular, the Cas9 endonuclease can have an amino acid sequence that is a variant or a fragment of any of the Cas9 endonuclease sequences of Genbank accession numbers KM099231.1 GL669193757; KM099232.1; GL669193761; or KM099233.1 GL669193765 or Cas9 amino acid sequence of PX330 or PX260 (Addgene, Cambridge, MA). The Cas9 nucleotide sequence can be modified to encode biologically active variants of Cas9, and these variants can have or can include, for example, an amino acid sequence that differs from a wild type Cas9 by virtue of containing one or more mutations (e.g., an addition, deletion, or substitution mutation or a combination of such mutations). One or more of the substitution mutations can be a substitution (e.g., a conservative amino acid substitution). For example, a biologically active variant of a Cas9 polypeptide can have an amino acid sequence with at least or about 50% sequence identity (e.g., at least or about 50%, 55%, 60%, 65%, 70%, 75%, 80%, 85%, 90%, 95%, 97%, 98%, or 99% sequence identity) to a wild type Cas9 polypeptide. Conservative amino acid substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine. The Cas9 nuclease sequence can be a mutated sequence. For example the Cas9 nuclease can be mutated in the conserved FiNH and RuvC domains, which are involved in strand specific cleavage. For example, an aspartate-to-alanine (D10A) mutation in the RuvC catalytic domain allows the Cas9 nickase mutant (Cas9n) to nick rather than cleave DNA to yield single-stranded breaks, and the subsequent preferential repair through HDR can potentially decrease the frequency of unwanted indel mutations from off-target double-stranded breaks. The polypeptides that are biologically active variants of a CRISPR-associated endonuclease can be characterized in terms of the extent to which their sequence is similar to or identical to the corresponding wild-type polypeptide. For example, the sequence of a biologically active variant can be at least or about 80% identical to corresponding residues in the wild-type polypeptide. For example, a biologically active variant of a CRISPR-associated endonuclease can have an amino acid sequence with at least or about 80% sequence identity (e.g., at least or about 85%, 90%, 95%, 97%, 98%, or 99% sequence identity) to a CRISPR-associated endonuclease or to a homolog or ortholog thereof. A biologically active variant of a CRISPR-associated endonuclease polypeptide will retain sufficient biological activity to be useful in the present methods. The biologically active variants will retain sufficient activity to function in targeted DNA cleavage. The biological activity can be assessed in ways known to one of ordinary skill in the art and includes, without limitation, in vitro cleavage assays or functional assays.

In particular, the population of alveolar macrophages obtained by the method of the present disclosure is genetically engineered to express a therapeutic nucleic acid of interest, which encodes a protein of interest or to inhibit the expression of a gene of interest and the production of the active form of the protein of interest.

The present disclosure will be further illustrated by the following figures and examples. However, these examples and figures should not be interpreted in any way as limiting the scope of the present disclosure.

### FIGURES:

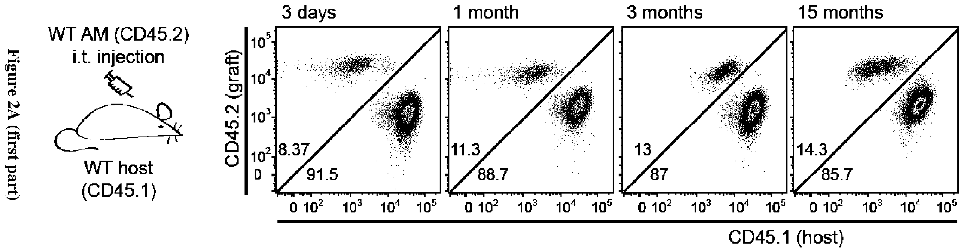

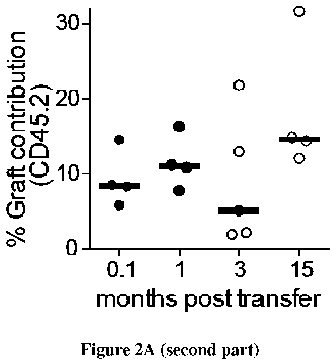

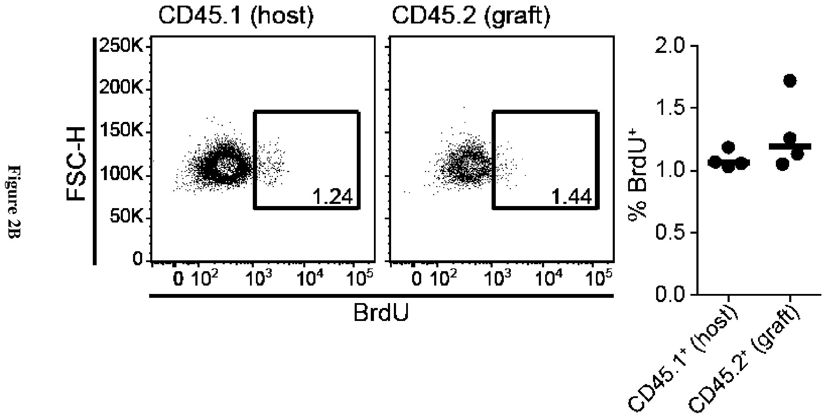

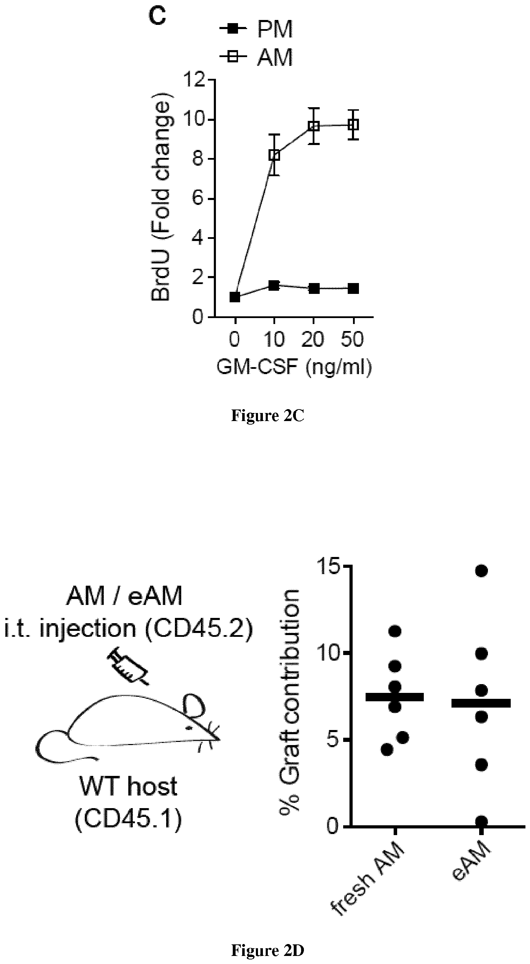

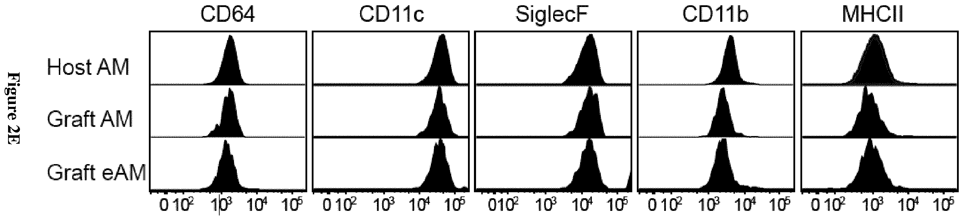

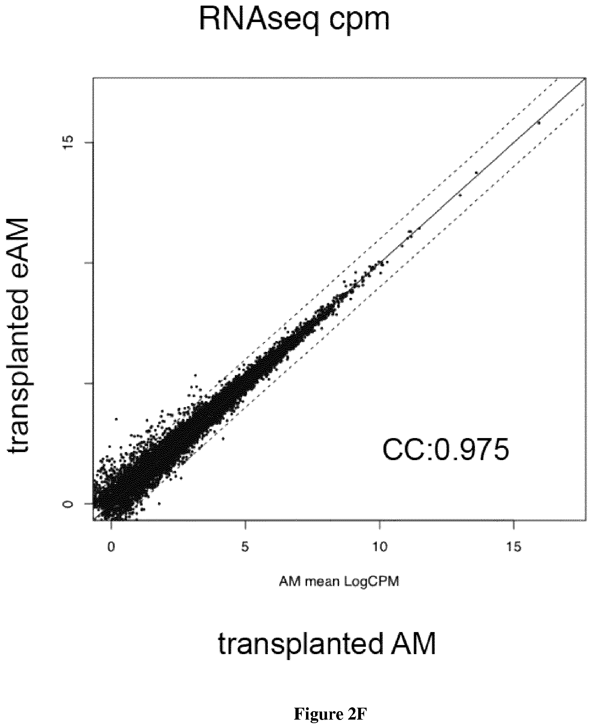

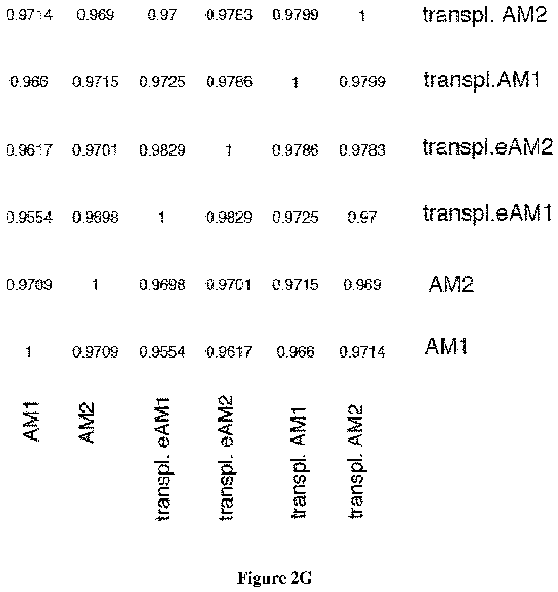

**Figure 1****.**
   A) Expression of MafB and cMaf relative to HPRT1, measured by RT-QPCR, in short-term cultures of bone marrow macrophages (BMM), peritoneal macrophages (PM) and alveolar macrophages (AM).
   B) Growth curve showing number of AM over time in liquid culture.
   C) Number of colony forming units (CFU) counted at day 21 per 10⁴ AM and PM plated in methocult medium after first plating, or after replating 10⁴ cells washed out from first plating (2^{nd} plating), or second plating (3^{rd} plating).
   D) Box plots showing average, inter-quartile and 5-95 percentile relative expression levels of self-renewal genes in Maf-DKO, WT BMM and AM, measured by nano-fluidic real-time PCR on Fluidigm array. ^{∗} p-value < 0.05; ^{∗∗} p-value < 0.01 based on an unpaired t-test.
   E) Aggregation plots showing average Chip-seq signals for PU.1 and P300 in Maf-DKO, WT BMM and AM for the self-renewal gene associated enhancers regions (total=88 regions).
   F) Pearson correlation matrix (PCA ranked log₂ read number) for PU.1 and P300 binding to self-renewal gene associated enhancers (total =88 regions) based on chip-seq data for Maf-DKO, WT BMM, and AM
**Figure 2****.** AM self-renew in vivo and in vitro and can integrate into the alveolar space upon pulmonary transplantation. (a,b) AM were isolated from lungs of WT (CD45.2) by BAL and were i.t. injected into WT (CD45.1) recipient (host) mice. (a) CD11c+SiglecF+ AM in cell suspensions generated from total lungs of recipient mice were analyzed by flow cytometry for CD45.1 and CD45.2 expression at indicated time point after injection. The median is indicated by bar (n= 4-5). Data are pooled from three independent experiment, (b) BrdU incorporation by host- and graft-derived AM was measured by flow cytometry 4/20 h post i.p. injection of 2 mg BrdU. The median is indicated by a bar (n = 4). Data are representative of two independent experiments. (c) In vitro analysis of BrdU incorporation by AM and PM 3 days after stimulation with GM-CSF and 18 h BrdU puls. Data are presented as fold increase over unstimulated cells and are representative of two independent experiments. Error bars represent SD of 3 technical replicates. (d,e) WT (CD45.1) recipient (host) mice were i.t. injected with either freshly isolated WT (CD45.2) AM or in vitro expanded eAM (CD45.2). (d) Contribution of graft-derived (CD45.2+) cells to the total CD1 1c+SiglecF+ AM population in total lung homogenates was analyzed by flow cytometry. The median is indicated by a bar (n = 6). (e) Surface marker expression of host-derived AM as determined by flow cytometry was compared to CD45.2+ grafted freshly isolated AM or eAM. Data are representative of two independent experiments. (f) Comparison of long term expanded and transplanted AM (eAM) to freshly transplanted AM global gene expression by scatterplot of RNAseq cpm showing the average of eAM and AM duplicates ; CC, spearman rho correlation coefficient. (g) Matrix of Spearman rho correlation coefficients of RNAseq global gene expression between individual transplanted eAM, transplanted AM and endogenous AM samples.

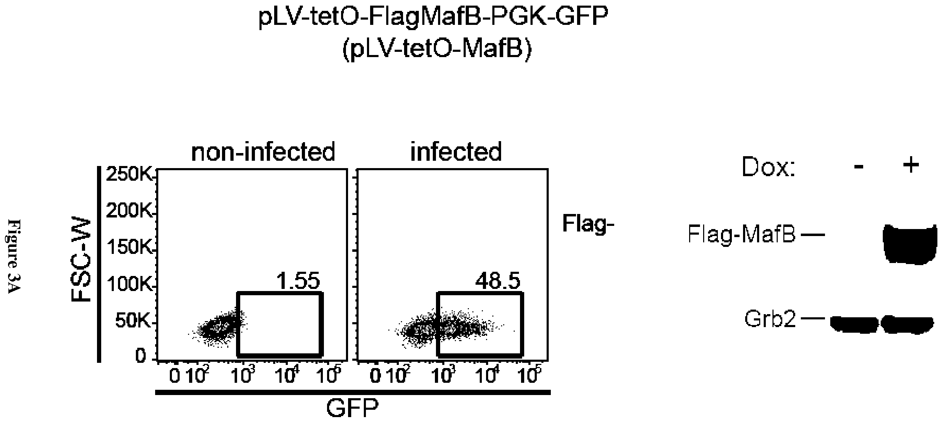

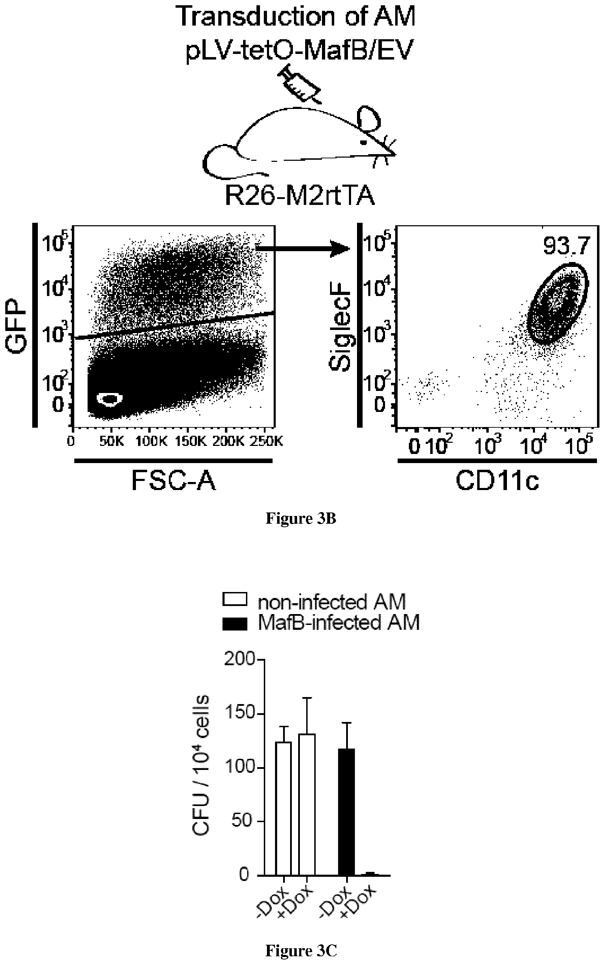

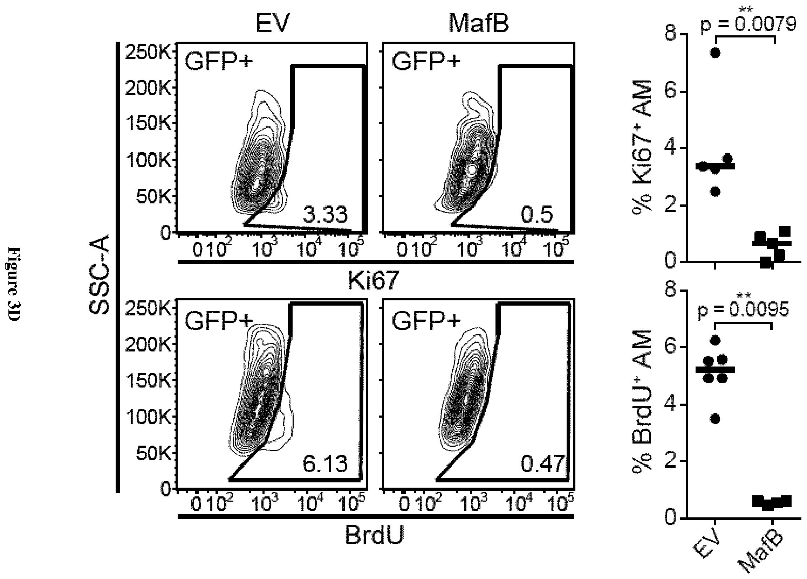

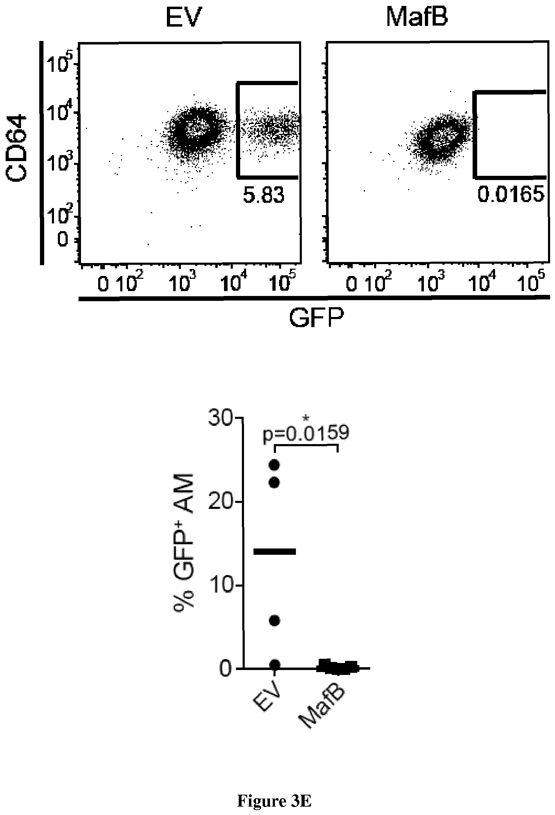

**Figure 3****.** Overexpression of MafB inhibits AM self-renewal in vitro and in vivo. (a) The lentiviral construct pLV-tetO-MafB was used for inducible overexpression of flag-tagged MafB. GFP expression of macrophages expressing the rtTA transactivator was analyzed by flow cytometry 48 h after in vitro infection with lentiviral particles generated the indicated viral construct. Flag-MafB expression after addition of DOX to the culture medium was detected by western blot in the same cells. Data are representative of two independent experiments. (b-f) R26-M2rtTA mice were i.t. injected with lentiviral particles generated from pLV-tetO-MafB or empty vector (EV) not containing the MafB sequence. (b) 7 days after injection, GFP expressing cells in total lung homogenates were identified by flow cytometry and were analyzed for expression of CD11c and SiglecF (n = 10). Data are representative of more than two independent experiments. (c) GFP+ and GFP- AM were sorted from total lung homogenates and used for CFA in the presence or absence of DOX in vitro. Quantification of colony formation represented as mean and SEM of a technical duplicate is shown in (c). Data are representative of three independent experiments, (d) Cytometric proliferation analysis of GFP+CD11c+SiglecF+ AM in total lung homogenates 7 days after virus injection and 3 days after induction of MafB expression by Ki67 expression (top) and BrdU incorporation after a 20 h pulse (bottom). The median is indicated by a bar (n = 4-6). Data shown are from two independent experiments and were analyzed by Mann-Whitney test. (e) Contribution of GFP+ cells to the total CD11c+SiglecF+ AM population in total lung homogenates was analyzed by flow cytometry 3 months after induction of MafB expression by DOX. The median is indicated by a bar (n = 4-5). Data shown are pooled from two independent experiments and were analyzed by Mann-Whitney test. (f) Cell death of GFP+ AM was determined by flow cytometry. The median is indicated by a bar (n = 6-7). Data are representative of three independent experiments and were analyzed by Mann-Whitney test.

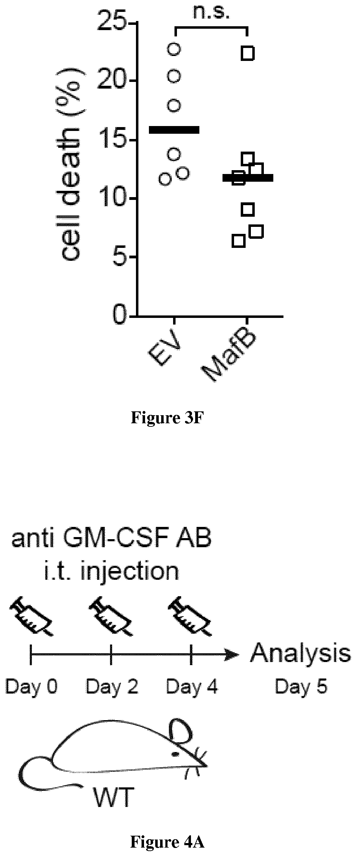

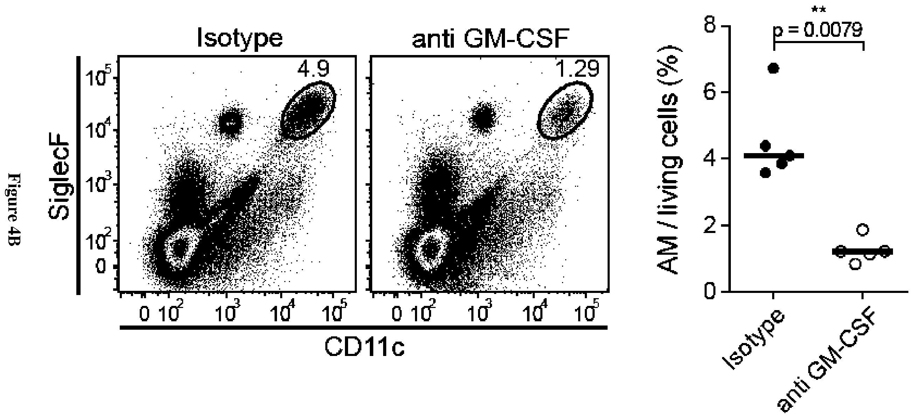

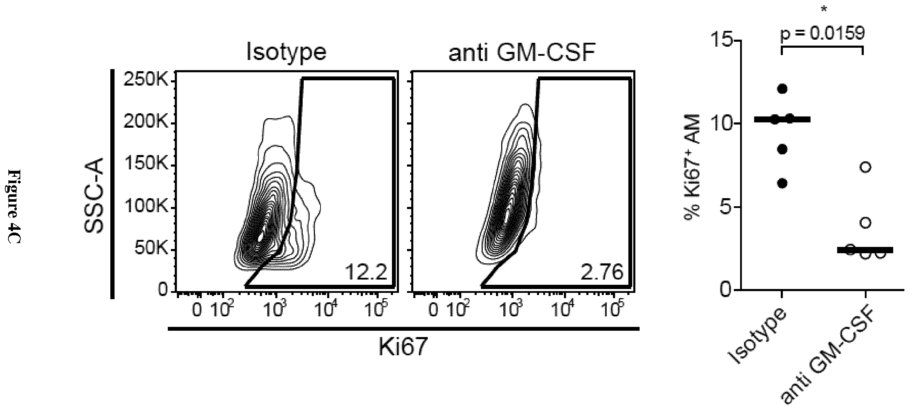

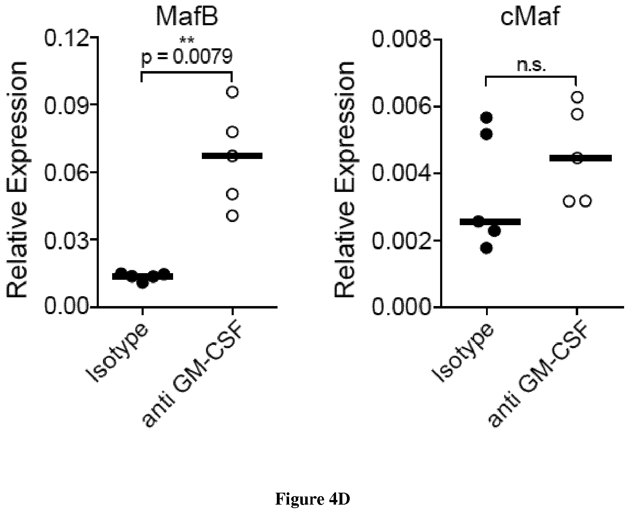

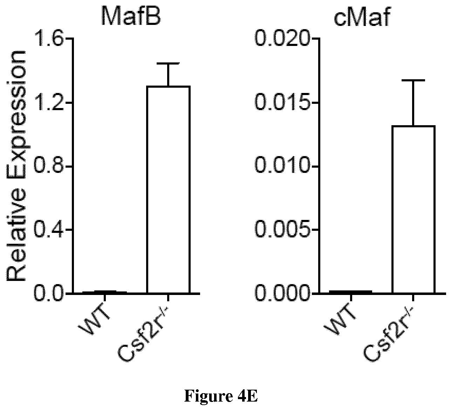

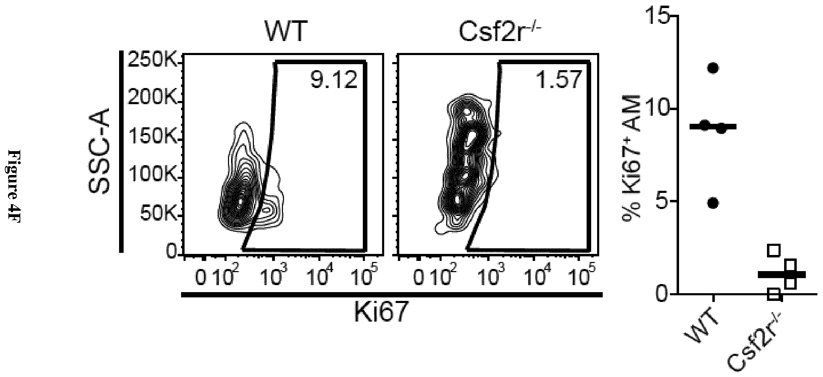

**Figure 4****.** MafB inhibits AM self-renewal in autoimmune and congenital PAP. (a-d) WT mice were i.t. injected with 100 ug anti-GM-CSF antibody or isotype control according to the scheme in (a) and were analyzed one day after the last injection. (b) Relative contribution of CD11c+Siglecf+ AM to total living cells was determined by flow cytometry. The median is indicated by a bar (n = 5). Data are representative of 2 independent experiments and were analyzed by Mann-Whitney test. (c) Cytometric proliferation analysis of CD11c+SiglecF+ AM in total lung homogenates by Ki67 expression. The median is indicated by a bar (n = 5). Data are representative of 2 independent experiments and were analyzed by Mann-Whitney test. (d) Analysis of MafB and cMaf expression by sorted AM as determined by qRT-PCR. The median is indicated by a bar (n = 5). Data are representative of 3 independent experiments and were analyzed by Mann-Whitney test. (e) Analysis for MafB and cMaf expression by AM isolated from BAL of WT and Csf2rb-/- mice as determined by qRT-PCR. Bars and error bars represent mean and SEM respectively (n = 5). Data are representative of 2 independent experiments. (f) Proliferation of F4/80+CD64+ macrophages in BAL fluid of WT and Csf2rb-/- mice was assessed by Ki67 expression by flow cytometry. The median is indicated by a bar (n = 4). Data are representative of 2 independent experiments and were analyzed by Mann-Whitney test.

### EXAMPLE 1:

### Material & Methods

For culture of alveolar macrophages, alveolar lavages were pooled from ten 1mL 37oC BAL washes (PBS, 2mM EDTA, 2% FBS (GE Healthcare) per mouse and stored on ice. RBC lysis was then performed at room temperature (RT) for 3 minutes (RBC Lysis Buffer, Invitrogen). Cells were plated at a density of at least 1.1 million cells per 10cm bacterial petri dish (Greiner Bio-One) or NUNC non treated 6well plates (>0,45-5^{∗}10⁶ per well) in complete medium (RPMI, 10 % FCS,1 % Pen/Strep, 1 % Pyruvate, 1 % Glutamate) supplemented with 1-2 % GM-CSF supernatant from J558L cells transfected with murine GM-CSF cDNA. Medium was partially changed every two-three days. Cells were replated only at confluency and only at least one day after medium change. To avoid cell death during detachment, cells were detached with Accutase (StemCells Technologies) and 2mM EGTA for 15 minutes at 37oC without agitation or pipetting and careful monitored under the microscope for the detachment process. Only after a large number of cells started to round up and float, cells were carefully detached with a p1000 pipettte.

### Results

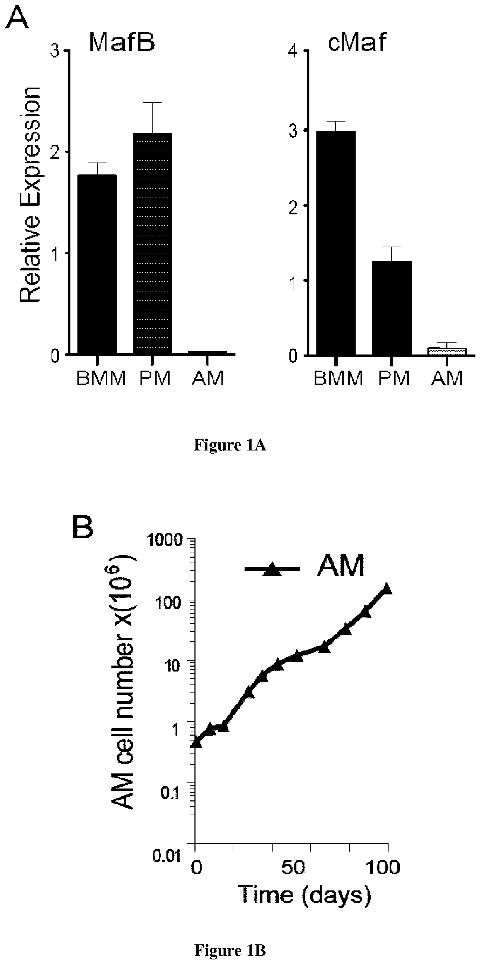

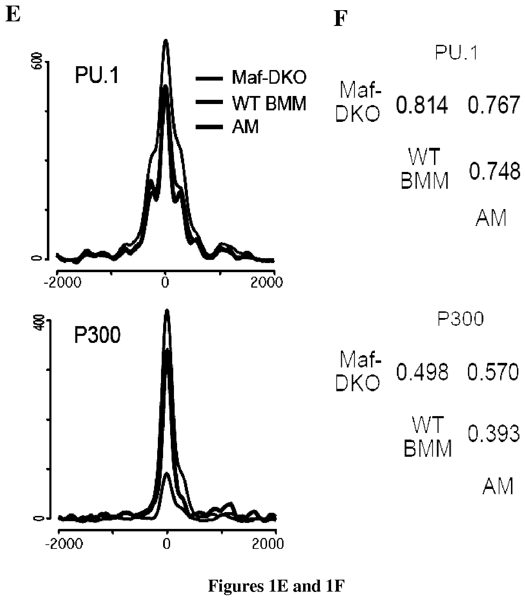

We have previously shown that inactivation of the two transcription factors MafB and c-Maf in macrophages (Maf-DKO macrophages) conferred the ability of extended, possibly unlimited self-renewal and expansion in culture to these cells (7). We further showed that this ability is due to the activation of a network of genes that is required for self-renewal in both embryonic stem cells and macrophages. We further investigated, whether our observations in Maf-DKO macrophages were directly relevant to the self-renewal capacity of genetically unmodified alveolar macrophages (AM). AM are a population of adult resident macrophages with the well-characterized ability to autonomously self-renew in vivo (4) and that in contrast to other characterized macrophage populations have the unique property to naturally express very low levels of MafB and cMaf(6). We confirmed these constitutively low Maf levels compared to other macrophage populations (Fig. 1A).

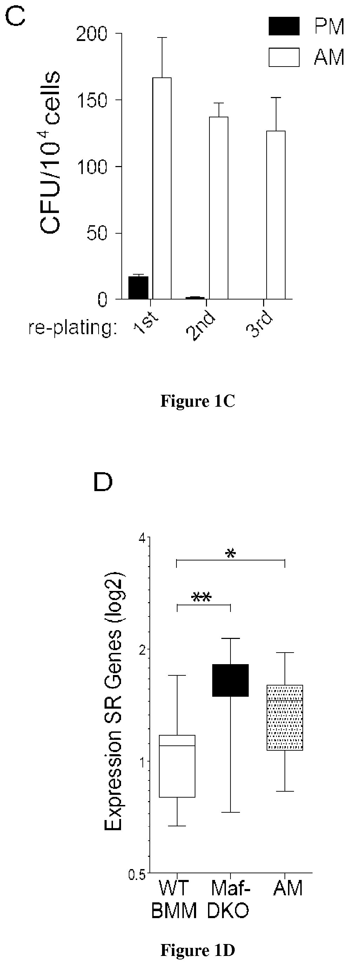

Surprisingly we could expand AM in long-term liquid culture for at least 100 days (Fig.1B) and serially replate AM but not PM in colony-forming assays in semi-solid medium without loss of replicative ability for at least three rounds of replating (Fig.1C). We could maintain AM in continuous liquid culture for at least 4 months through at least 16 passage numbers and expand their numbers to at least 10 population doublings. Cultered AM could be frozen in liquid nitrogen and re-thawed without loss of replicative capacity. Cultured alveolar macrophages also expressed generally increased levels of self-renewing network genes (Fig.1D). Furthermore, Chip-Seq analysis of epigenetic enhancer marks at the SR gene associated enhancer regions showed comparable binding of the macrophage lineage defining PU.1 transcription factor between AM, Maf-DKO and WT BM macrophages, but an enrichment of the activation mark p300 in AM similar to Maf-DKO macrophages (Fig. 1 E, data not shown). Statistical analysis confirmed a high correlation for PU.1 binding across all three populations but the highest correlation for p300 binding between AM and Maf-DKO macrophages (Fig. IF). Together these results demonstrate that similar to experimental inactivation natural low levels of MafB and c-Maf levels occurring in alveolar macrophages activate a set of poised macrophage-specific enhancers and a self-renewal gene network that permits the extended culture and possibly unlimited self-renewal of alveolar macrophages.

### EXAMPLE 2:

Here the inventors showed that in culture expanded alveolar macrophages (eAM) can be transplanted intra-tracheal, integrated into the endogenous AM population and continue to proliferate in vivo like recipient AM

They further demonstrated that eAM are maintained long term in graft recipient, for at least 15 months (3/4 of the life span of a mouse).

It is also shown that eAM maintain AM identity even after long term culture (>4months) and subsequent long term transplantation into recipients for > 7 months.

### Material & Methods

**Mice.** All transgenic mice used in this study have a C57Bl/6 background (>7 backcrosses) and were between 6 and 14 weeks old, if not stated otherwise. CD45.1 and CD45.2 C57Bl/6 mice were obtained from Charles River. Csf2rb1-/- mice (Shibata, Y. et al. GM-CSF regulates alveolar macrophage differentiation and innate immunity in the lung through PU.1. Immunity 15, 557-567 (2001)) were provided by Prof. Dr. Melanie Greter (University of Zurich). Rosa26-M2rtTA mice (Happle, C. et al. Pulmonary transplantation of macrophage progenitors as effective and long-lasting therapy for hereditary pulmonary alveolar proteinosis. Sci Transl Med 6, 250ra113 (2014)) were provided by The Jackson Laboratory. Csf2-/- mice (Dranoff, G. et al. Involvement of granulocyte-macrophage colony-stimulating factor in pulmonary homeostasis. Science 264, 713-716 (1994)) were provided by Prof. Dr. Irmgard Forster (University of Bonn).

For i.t. injections, mice were anesthetized by isoflurane inhalation and were placed on their back. The tongue was gently pulled out and fixed with a forceps, while mice were injected with 50-100 ul into the trachea using a 1 ml syringe and a blunt 22G feeding needle. The fluid was inhaled into the lungs by subsequent respiratory efforts. Mice were observed while recovering from anesthesia to ensure retention of the administered fluid and then returned to their cages for routine care and handling.

For i.t. cell injections, 100 ul of PBS containing 1^{∗}10^6 cells were injected into each mouse. I.t. injection of lentiviral particles is described below. For neutralization of GM-CSF, 100 ul of PBS containing 100 ug of monoclonal rat anti-mouse IgG2a antibody were used for each injection (neutralizing antibody against GM-CSF (clone 22E9) or isotype control antibody of irrelevant specificity; provided by Dr. John A. Hamilton (Vlahos, R. et al. Neutralizing granulocyte/macrophage colony-stimulating factor inhibits cigarette smoke-induced lung inflammation. Am J Respir Crit Care Med 182, 34-40 (2010))). Every i.t. injections of recombinant M-CSF (rhM-CSF, Novartis) contained 20 ug of M-CSF in 100 ul of PBS.

For inhibition of the M-CSF-R in vivo, 100 ug of GW2580 (Calbiochem/Merck millipore) in 200 ul of PBS were i.p. injected twice per day starting 12 h prior to injection of cells. On day 0, 1^{∗}10^6 freshly isolated CD45.1+ AM were resuspended in 100 ul PBS containing 5 uM GW2580 and i.t. injected into recipient mice. I.p. injections of GW2580 were continued until day 2 after cell transfer (7 i.p. injections of GW2580 in total) and CD45.1+ AM were sorted from BAL fluid on day3 for gene expression analysis.

Bone marrow chimeric mice were generated by lethal irradiation of 7-10 week old WT CD45.1 host mice that were subsequently reconstituted by i.v. injection of 1^{∗} 10^6 BM cells isolated from donor mice. Donor BM was isolated from femurs and tibias of chimeric CD45.1 donor mice, that had been reconstituted with 4 ^{∗} 10^6 fetal liver cells from WT and Maf-DKO E14.5 CD45.2 embryos. CD45.2+ AM from the lungs of the chimeras were isolated for in vitro CFA experiments after at least 10 weeks after reconstitution.

Doxycycline induced gene expression in vivo was induced 4 -8 days after virus injection by providing drinking water containing 2 g/l DOX and 2 % sucrose as well as DOX containing food (Plexx B.V., ref. S3888, Doxycycline (200 mg/kg) to the mice ad libitum. Drinking water was replaced every 2 days.

All mouse experiments were performed under specific pathogen-free conditions and according to the protocols approved by the animal ethics committee of Marseille (France). Cell and organ isolation from mice were performed according to the protocols approved by the animal ethics committee of Marseille (France) and the Max-Delbrück-Centrum Berlin (Germany).

**Cell Culture.** For isolating alveolar macrophages, bronchoalveolar lavage (BAL) was performed as decribed (Soucie, E.L. et al. Lineage-specific enhancers activate self-renewal genes in macrophages and embryonic stem cells. Science (2016)) using 1 ml of wash buffer (PBS, 2mM EDTA, 2% FCS) for each wash step and pooling up to 10 wahes per mouse. BAL fluid was collected on ice. RBC lysis was then performed at room temperature (RT) for 3 minutes (RBC Lysis Buffer, Invitrogen) and cells were filtered through a 70 um mesh before further processing. For expansion, cells were plated at a density of 1.1 million cells per 10 cm petri dish in complete medium (RPMI, 10 % FCS,1 % Pen/Strep, 1 % Pyruvate, 1 % Glutamate) supplemented with 1 % GM-CSF supernatant from J558L cells transfected with murine GM-CSF cDNA. For detaching AM, cells were washed with PBS and incubated with accutase (Merck Millipore) supplemented with 1 mM EGTA (Sigma) for 15 minutes before collection of detached cells.

Peritoneal macrophages were collected by peritoneal washout using 10 mL PBS containing 2 mM EDTA (Sigma). Red cell lysis was performed (RBC Lysis Buffer, Invitrogen) and cells were filtered through a 70 um mesh before further processing. Medium changes occurred every 3-4 days.

Maf-DKO macrophages were grown sub-confluently in macrophage growth medium (Dulbecco's modified medium (DMEM) with 20% L-cell sup, 1% sodium pyruvate and 1% L-Glutamate) as described (Aziz, A., Soucie, E., Sarrazin, S. & Sieweke, M.H. MafB/c-Maf deficiency enables self-renewal of differentiated functional macrophages. Science 326, 867-871 (2009)).

WT BMM macrophages were differentiated from total mouse bone marrow for 15 days in macrophage growth medium, selecting for adherent cells every 4 days.

For co-injection of WT and Maf-DKO macrophages, cultured WT BMM and Maf-DKO macrophages were detached and stained with 2 uM of CFSE or cell trace violet (Thermo) according to manufacturer's instructions. After mixing cells with CFSE and CTV staining, the exact pre-injection ratio of CTV+/CFSE+ cells was determined by flow cytometry.

**Preparation of total lung homogenates and BAL fluid for flow cytometry analysis.** For preparing single cell suspension from total lung tissue, mice were killed by cervical dislocation and perfused with PBS before collecting the tissue. Lung tissue was macerated and incubated with 1 mg/ml collagenase-2 (Worthington) and 0.15 mg/ml DNase1 (Sigma) at 37°C for 30 min during constant agitation. Resulting cell suspension was filtered through a 70 um mesh and erythrocytes were removed by RBC lysis (RBC Lysis Buffer, Invitrogen) before proceeding to the flow cytometry staining protocol.

BAL was performed as described above. After RBC lysis (RBC Lysis Buffer, Invitrogen)and filtration cells could be processed according to the flow cytometry staining protocol.

**Flow cytometry.** Cell suspension was treated with Zombie Aqua Fixable Viability Kit (BioLegend) according to the manufacturer's instructions to label dead cells. Before proceeding with antibody staining, cells were pre-incubated with Fc receptor blocking antibody (clone 2.4 G2, PD Pharmingen) for 20 minutes at 4°C. For antibody staining, cells were incubated with antibody cocktail for 20 minutes at 4°C. Cells were acquired on FACSCanto, LSRII and LSRFortessa systems (BD) and analyzed with Flowjo software (TreeStar). The following antibodies were used for staining cells: Anti-CD11b (clone M1/70, eBioscience), anti-CD11c (clone N418, eBioscience), anti-F4/80 (clone BM8, eBioscience), anti-MHCII (clone M5/114.15.2, eBioscience), anti-CD45.1 (clone A20, eBioscience), anti-CD45.2 (clone 104, eBioscience), anti-B220 (clone RA3-6B2, eBioscience), anti-CD115 (clone AFS98, eBioscience), anti-CD64 (clone X54-5/7.1, Biolegend), anti-SiglecF (clone E50-2440, BD Pharmingen), anti-Ki67 (clone B56, BD Pharmingen and clone SolA15, eBioscience), anti-BrdU (clone MoBU-1, Life technologies).

**Cell sorting.** For gene expression analysis of subsets of total AM of experimental mice we sorted single living AM from total lung homogenates and BAL fluid according to the marker combination indicated in the respective figure legend on a BD FACSAria III machine. Cells were sorted directly into lysis buffer for RNA extraction.

**Cell cycle analysis.** In vitro proliferation of macrophages was assessed using the Cell Proliferation ELISA (BrdU, colorimetric; Roche) according to manufacturer's instructions. In brief, 10-20 thousand freshly islolated macrophages were plated per 96 well in a tissue culture dish in complete medium (DMEM, 10 % FCS,1 % Pen/Strep, 1 % Pyruvate, 1 % Glutamate). After macrophages had attached to the plastic, plating medium was replaced with complete medium containing the respective cytokine at indicated concentration. On day 2 after stimulation, BrdU was added to the culture and BrdU incorporation was assessed on day 3.

Ex vivo analysis of BrdU incorporation by AM was assessed 4 or 20 h (as specified in figure legends) after intraperitoneal injection of mice with 2 mg BrdU (Sigma) by flow cytometry using BrdU Flow kit (BD Pharmingen) according to manufacturer's instructions with minor modifications, including extended DNaseI digestion (90 minutes at 37°C) and intracellular staining (30-60 minutes). For BrdU staining a different monoclonal antibody than provided in the kit was used (Thermo Fischer Scientific, clone MoBU-1). For Ki67 staining, cells were treated as for BrdU staining but stained with an anti-Ki67 antibody instead (clone B56, BD Pharmingen or clone SolA15, eBioscience).

**Lentivirus production and transduction.** The lentiviral construct pLV-TetO-FlagMafB-PGK-GFP was generated by cloning an insert PCR product containing FlagMafB-PGK-GFP into the EcoRI site of pLV-TetO-Klf4 (Addgene #19764, Klf4 cDNA was removed by EcoRI digestion). pRetroX-Tight-GFP containing flag-tagged MafB (Soucie, E.L. et al. Lineage-specific enhancers activate self-renewal genes in macrophages and embryonic stem cells. Science (2016)) served as a template for generating the insert by two sequential PCRs. Forward-Primer-Flag-MafB and Reverse-Primer-MafB were used to amplify Flag-tagged MafB. Forward-Primer-PGK-GFP and Reverse-Primer-GFP-PGK were used to amplify PGK-GFP. In a second step, the resulting overlapping PCR products from these reactions were used as templates for a second PCR using Forward Primer Flag-MafB and Reverse Primer GFP-PGK resulting in the desired insert. This two step overlapping PCR strategy was necessary to mutate a EcoR1 site between MafB and the PGK promoter.

Lentiviral vector particles were produced at the Centre International de Recherche en Infectiologie (CIRI) U1111/UMR5308 Inserm-CNRS-UCBL - ENS de Lyon (Lyon, France) by tri-transfection of plasmids harboring the pack-aging construct, the transfer vector (Amit, I. et al. Unbiased reconstruction of a mammalian transcriptional network mediating pathogen responses. Science 326, 257-263 (2009)) and the envelope-expressing construct into producer cells using calcium chloride methodology. Viral supernatants were concentrated by ultracentrifugation and viral particles were resuspended in PBS resulting in viral titers of > 10⁷ pfu.

For in vivo transduction of AM 50 - 100 ul of concentrated viral particles were i.t. injected into recipient mice. 4-8 days after virus injection, gene expression was induced by providing mice with drinking water containing DOX (2 g/l; water was also supplemented with 2% sucrose; Dox containing water was changed every two days) and DOX containing food (Plexx B.V., ref. S3888, Doxycycline (200 mg/kg).

For in vitro transduction, 10 ul of concentrated viral particles were added to Maf-DKO macrophages expressing rtTA (Soucie, E.L. et al. Lineage-specific enhancers activate self-renewal genes in macrophages and embryonic stem cells. Science (2016).) seeded in a 12 dish containing 350 ul of medium. Medium was changed after 4 h incubation at 37°C.

### Western blot.

Protein were extracted by direct lysing in SDS sample-buffer (50mM Tris-HCl, pH6,8; 4% SDS, 20% Glycerol, 10% 2-mercoptoethanol (beta-ME; Bromophenol Blue). Samples were transfered to a QIAshredder column and centrifuged at maximum speed for 2 minutes, in order to completely disrupt the cells. Proteins were denatured by boiling for 5 minutes at 100dC. Proteins were separated on 10% SDS-PAGE and then transferred to Protran 0,2 µm nitrocellulose transfer membrane (Schleicher & Schuell). at 2.5mA/cm^2 using 25mM Tris base, 192 mM Glycine, and 20% v/v methanol as transfer buffer. Membrane was blocked overnight with TBS-Tween (13.6mM NaCl, 20mM Tris base, ImM EDTA; pH adjusted to 7.8 with HCl 1M; 0.05% Tween 20) containing 5% skimmed milk powder (blocking solution). Primary antibodies were diluted in blocking buffer (1:4000 anti-Flag Sigma, 1:1000 anti-GRB2 Abcam) and incubated for 1 hour at RT with constant agitation. Secondary antibodies were diluted in blocking buffer (mouse anti-IgG and rabbit anti-IgG ab both coubled to HRP, Santacruz) and incubated for 45 minutes. Chemiluminescence was aquired on automatic revelation WB machine (Thermo Scientific).

**Colony froming assay.** AM were plated at 10,000 cells per 1 mL of Methocult M3231 (StemCell Technologies) containing 100 ng/ml of rGM-CSF (Peprotech) or rM-CSF (Peprotech) if not indicated otherwise. Colonies were counted after 3 weeks.

**Gene Expression Analysis.** For gene expression analysis, macrophages were isolated from BAL fluid of WT or Csf2rb-/- mice through adherence to plastic for 2 hours. RNA was either extracted directly or after in vitro stimulation as specified in the figure legends. Total RNA was isolated from cells by using RNeasey mini kit (Qiagen) and Qiashredder columns (Qiagen) according to manufacturer's instructions. On column DNA digestion was performed. 100 ng of total RNA was used for reverse transcription to cDNA using SuperScript III RT (Thermo), Oligo(dT) primers (Thermo) and RNaseOUT (Thermo). Quantitative PCR was performed using Fast SYBR Green Master Mix (Thermo).

### RNAseq analysis

For gene expression analysis of endogenous or transplanted AM we sorted single living AM from total lung homogenates according to the marker combination indicated in the respective figure legend on a BD FACSAria III machine. Cells were sorted directly into lysis buffer for RNA extraction. We analysed biological replicates from 10-30.000 cells of indicated populations with TruSeq total RNA kit for sequencing on a HiSeq Illumina sequencer. Adaptor and quality (Phred Score > 20) trimmed reads were aligned on the mouse genome version mm9 using Tophat2 suite and then converted to count data using HTSeq-count. Transcripts having low counts were excluded and read counts were normalized using the Trimmed of Mean of M value approach. In corridor plot analysis gene expressions were normalized by millions of aligned reads (CPM). Spearman rank correlation (CC) was either calculated on a mean of replicates for each condition or for each pairwise comparison.

**BAL fluid analysis.** 4 weeks after transplantation of WT or Maf-DKO macrophages, BAL fluid from Csf2-/- mice was collected by performing 5 washes with 1 ml of PBS. Protein concentration was determined using Bradford Protein Assay (Biorad). BAL turbidity was determined by measuring the OD at 600 nm wavelength.

### Results

### Transplanted and in vitro expanded AM can be maintened long term in vivo

Self-renewal of AM in vivo has been established through experiments employing genetic lineage tracing and parabiosis (Yona, S. et al. Fate Mapping Reveals Origins and Dynamics of Monocytes and Tissue Macrophages under Homeostasis. Immunity 38, 79-91 (2013)) (Hashimoto, D. et al. Tissue-resident macrophages self-maintain locally throughout adult life with minimal contribution from circulating monocytes. Immunity 38, 792-804 (2013)) (Guilliams, M. et al. Alveolar macrophages develop from fetal monocytes that differentiate into long-lived cells in the first week of life via GM-CSF. J Exp Med 210, 1977-1992 (2013)). To assess whether transplanted AM can be maintained long term, we transferred freshly isolated WT AM (CD45.2) into the lungs of WT mice (CD45.1) by intratracheal (i.t.) injection and analyzed the lungs of the host mice at different time points after transfer by flow cytometry (Fig. 2a). AM were identified as CD11c+SiglecF+ cells (data not shown) and we found that around 10 % of all AM were graft-derived at 3 days after transfer. Importantly, although, i.t. injection efficiency is very variable, this level of contribution of graft-derived AM to the total AM population remained stable over the observation period of 15 months without being diluted or outcompeted by host-derived cells (Fig. 2a). Accordingly, we found no difference in the proliferation rate of graft- and host-derived AM as assessed by BrdU incorporation (Fig. 2b).

AM express exceptionally low levels of MafB and cMaf as compared to other macrophage populations including peritoneal macrophages (PM) (Fig.1A). Previous reports have demonstrated extended self-renewal of macrophages lacking MafB and cMaf (Aziz, A., Soucie, E., Sarrazin, S. & Sieweke, M.H. MafB/c-Maf deficiency enables self-renewal of differentiated functional macrophages. Science 326, 867-871 (2009)) as well as transient proliferation of AM in response to GM-CSF (Chen, B.D., Mueller, M. & Chou, T.H. Role of granulocyte/macrophage colony-stimulating factor in the regulation of murine alveolar macrophage proliferation and differentiation. J Immunol 141, 139-144 (1988)) (Akagawa, K.S., Kamoshita, K. & Tokunaga, T. Effects of granulocyte-macrophage colony-stimulating factor and colony-stimulating factor-1 on the proliferation and differentiation of murine alveolar macrophages. J Immunol 141, 3383-3390 (1988).) in culture. We found that cultured AM but not PM incorprorated BrdU and expressed the mitotic marker phospho-histone H3 (pHH3) in response to GM-CSF (Fig. 2c, data not shown). Importantly and surpisingly, however,, AM could be serially replated in colony-forming assays and we were able to expand AM for at least 10 population doublings in long-term liquid culture in the presence of GM-CSF for several months (Soucie et al. Science 2016; Fig.1B,C, data not shown). In vitro expanded AM (eAM) displayed a surface marker phenotype similar to freshly isolated AM with the characteristic high expression of SiglecF and CDllc (data not shown). To test whether eAM retain their capacity to integrate into the lung tissue environment after multiple passages in vitro we i.t. transferred AM that had been expanded for over 4 months (eAM) in comparison to freshly isolated AM (both CD45.2) into the lungs of WT mice (CD45.1) and found that 20 weeks after transfer grafted eAM integrated into the host AM population with similar efficiency as freshly isolated grafted AM (Fig. 2d). The in vivo surface marker expression phenotype of the grafted eAM and freshly isolated AM was indistinguishable from host AM at 2 months post transfer as defined by surface marker expression (Fig. 2e). Furthermore RNAseq analysis revealed that the gene expression profile of transplanted AM that had been expanded in culture for at least 4 months (eAM) were nearly identical to freshly transplanted AM 7 months after transplantation as well as to endogenous AM of the transplanted mice (Fig.2f,g). Together, these data demonstrate that AM amplified long term in culture integrate into normal mcrophage populations of lung aveoli tissue upon intra-tracheal transfer, are maintained long term in vivo for at least up to ¾ of the life span of the recipient and maintain normal AM identity after transplantation.

### MafB expression abrogates AM self-renewal

To test whether self-renewal and maintenance of AM was depended on low expression of the transcription factors MafB we constructed a lentiviral vector encoding for Flag-tagged MafB under the control of a tetracycline-inducible promoter and GFP under the control of a constitutive PGK promoter and confirmed functionality by lentiviral transduction of macrophages expressing the reverse tetracycline-controlled transactivator (rtTA) (Fig. 3a). We performed in vivo transduction of AM by i.t. injection of these lentiviral particles into transgenic R26-M2rtTA mice, which express the reverse tetracycline-controlled transactivator from the ROSA26 locus (Hochedlinger, K., Yamada, Y., Beard, C. & Jaenisch, R. Ectopic expression of Oct-4 blocks progenitor-cell differentiation and causes dysplasia in epithelial tissues. Cell 121, 465-477 (2005)), resulting in GFP expression by infected AM (Fig. 3b). To test whether MafB expression inhibits AM self-renewal we isolated infected GFP+ and non-infected GFP- AM from the lungs of injected R26-M2rtTA mice by cell sorting, and assessed self-renewal by colony forming assay (CFA) (Fig. 3c). In the absence of doxycycline (DOX), colony formation of infected GFP+ AM was equal to non-infected AM in the presence or absence of DOX, whereas colony formation of GFP+ AM was blocked upon MafB expression (Fig. 3c). Absence of colonies was not due to cell death as individual living cells but no colonies could be found in the cultures (data not shown). To address whether MafB also suppresses self-renewal of AM in vivo we instilled lentiviral particles encoding for inducible MafB or empty vector (EV) into the lungs of R26-M2rtTA mice and induced MafB expression by supplementing food and drinking water with DOX. Cytometric analysis of AM proliferation by Ki67 staining and BrdU incorporation revealed that proliferation of GFP+ AM was specifically abrogated in the presence of MafB, whereas EV transduced or uninfected AM showed unaltered levels of steady state proliferation (Fig. 3d and data notshown). Additionally, we analyzed long-term contribution of GFP+ cells to the total AM population after 3 months of DOX-treatment and found no persisting AM infected with MafB lentiviruses whereas up to 25 % of AM were GFP+ in the case of EV infection (Fig. 3e). Disappearance of MafB infected AM depended on MafB expression, as GFP+ AM persisted in the absence of DOX-treatment (data not shown). Loss of MafB expressing AM was not due to cell death as no increase in dead cells could be detect among AM expressing MafB as compared to EV (Fig. 3f). Also, non-cell intrinsic effects were unlikely, as almost all lentivirus-infected cells in the lung were AM (Fig. 3b). Overall, these results demonstrate that MafB expression blocks AM self-renewal in vitro and in vivo.

### AM upregulate MafB and cMaf during PAP

We also observed that inhibition of AM self-renewal by MafB also occured under pathophysiological conditions. Pulmonary alveolar proteinosis (PAP) is a disease caused by malfunction of AM leading to the accumulation of surfactant in the lung. The vast majority of PAP patients suffer from the acquired form of the disease caused by autoantibodies against GM-CSF (Trapnell, B.C., Carey, B.C., Uchida, K. & Suzuki, T. Pulmonary alveolar proteinosis, a primary immunodeficiency of impaired GM-CSF stimulation of macrophages. Curr Opin Immunol 21, 514-521 (2009)), which we decided to mimic experimentally. To this end, we treated WT mice with i.t. injections of monoclonal antibodies against GM-CSF (Fig. 4a) and analyzed AM number, proliferation and MafB expression 1 day after the last injection. Neutralization of GM-CSF led to a decreased frequency of AM in the lungs of treated mice in comparison to isotype-treated controls (Fig. 4b). Importantly, we also observed a decrease in AM proliferation (Fig. 4c), which was accompanied by upregulation of MafB (Fig. 4d), arguing for an inhibition of AM self-renewal by MafB in autoimmune PAP. The role of cMaf seems to be less prominent in this setting, as the expression levels were much lower and no significant upregulation could be detected (Fig. 4d).

After identifying a role of MafB in the regulation of AM proliferation during autoimmune PAP, we also analyzed AM proliferation in Csf2rb-/- mice, which represent a model for congenital PAP. Importantly, MafB and to a lesser extend cMaf were upregulated in Csf2rb-/- AM (Fig. 4e), and proliferation was inhibited (Fig. 4f), indicating that MafB blocks self-renewal of AM in congenital PAP. Taken together, we have shown that inhibition of AM self-renewal during acquired and congenital PAP pathogenesis is associated with increased MafB expression. This underlines the pathophysiological relevance of MafB-mediated regulation of AM self-renewal. It also indiacates that the inactivation, deletion or inhibition of MafB in AM would improve AM self-renewal in disease settings, where MafB is abnormally up-regulated and blocks AM self renewal. We are performing experiments showing that AM, in which MafB has been inactivated have an improved self renewal capacity after transplantation into models of PAP. Together our data demonstrate that MafB inactivation in transplanted AM can have therapeutic benefit.

### REFERENCES:

Throughout this application, various references describe the state of the art to which the present disclosure pertains.
1. S. J. Jenkins et al., Local macrophage proliferation, rather than recruitment from the blood, is a signature of TH2 inflammation. Science 332, 1284 (Jun 10, 2011).
2. C. Schulz et al., A lineage of myeloid cells independent of Myb and hematopoietic stem cells. Science 336, 86 (Apr 6, 2012).
3. S. Yona et al., Fate mapping reveals origins and dynamics of monocytes and tissue macrophages under homeostasis. Immunity 38, 79 (Jan 24, 2013).
4. D. Hashimoto et al., Tissue-Resident Macrophages Self-Maintain Locally throughout Adult Life with Minimal Contribution from Circulating Monocytes. Immunity 38, 792 (Apr 18, 2013).
5. M. H. Sieweke, J. E. Allen, Beyond stem cells: self-renewal of differentiated macrophages. Science 342, 1242974 (Nov 22, 2013).
6. E. L. Gautier et al., Gene-expression profiles and transcriptional regulatory pathways that underlie the identity and diversity of mouse tissue macrophages. Nat Immunol 13, 1118 (Sep 30, 2012).
7. A. Aziz, E. Soucie, S. Sarrazin, M. H. Sieweke, MafB/c-Maf deficiency enables self-renewal of differentiated functional macrophages. Science 326, 867 (Nov 6, 2009).
8. G. Fejer et al., Nontransformed, GM-CSF-dependent macrophage lines are a unique model to study tissue macrophage functions. Proc Natl Acad Sci U S A 110, E2191 (Jun 11, 2013).

## Claims

1. A method of expanding a population of alveolar macrophages in a long term liquid culture comprising culturing for at least 100 days the population of alveolar macrophages in a culture medium supplemented with an amount of GM-CSF.

2. Use of the method of claim 1 for preparing a pharmaceutical composition comprising at least 1 ×10⁹ of alveolar macrophages.

## Patentansprüche

1. Verfahren zum Erweitern einer Population von alveolären Makrophagen in einer Langzeit-Flüssigkultur, umfassend das Kultivieren der Population von alveolären Makrophagen für mindestens 100 Tage in einem mit einer Menge an GM-CSF supplementierten Kulturmedium.

2. Verwendung des Verfahrens nach Anspruch 1 zur Herstellung einer pharmazeutischen Zusammensetzung, umfassend mindestens 1 × 10⁹ alveoläre Makrophagen.

## Revendications

1. Procédé d'amplification d'une population de macrophages alvéolaires dans une culture liquide à long terme comprenant une culture pendant au moins 100 jours de la population de macrophages alvéolaires dans un milieu de culture complété avec une quantité de GM-CSF.

2. Utilisation du procédé selon la revendication 1 pour préparer une composition pharmaceutique comprenant au moins 1 × 10⁹ de macrophages alvéolaires.
